# FASCICULE DE BREVET EUROPEEN

(11) **EP 0 504 040 B1**
(45) Date de publication et mention de la délivrance du brevet: **14.06.1995**
(21) Numéro de dépôt: 92400626.5
(22) Date de dépôt: 11.03.1992
(51) Int. Cl.: A61K 7/06, A61K 7/48

(54) **Utilisation de la lactoferrine pour la protection de la kératine des cheveux contre les agressions atmosphériques, notamment la lumière, et procédé de protection des cheveux utilisant ce composé**
Verwendung von Lactoferrin zum Schutz des Haarkeratins gegenüber atmosphärischen Einflüssen, insbesondere Licht, und Verfahren zum Schützen der Haare, das diese Verbindung verwendet
Use of lactoferrin to protect hair keratin from atmospheric aggressions, particularly light, and process for protecting the hair using this compound

(30) Priorité: 13.03.1991 FR 9103018
(43) Date de publication de la demande: 16.09.1992
(73) Titulaire: L'OREAL, 75008 Paris (FR)
(72) Inventeur: Dubief, Claude, F-78150 Le Chesnay (FR)
(74) Mandataire: Casalonga, Axel

(56) Documents cités:
- EP-A- 0 223 257
- FR-A- 2 287 899
- FR-A- 2 596 986
- FR-A- 2 620 024
- FR-A- 2 641 696

## Description

La présente invention a pour objet l'utilisation de la lactoferrine en tant qu'agent de protection de la kératine des cheveux contre les agressions atmosphériques, et en particulier la lumière, et un procédé de protection des cheveux contre les agressions atmosphériques, et en particulier contre la lumière.

On sait depuis longtemps que la lumière agresse la kératine des cheveux. De nombreuses publications divulguent que la lumière naturelle détruit certains aminoacides des cheveux et qu'en altérant la fibre capillaire, elle en diminue les propriétés mécaniques comme la résistance à la traction, la charge de rupture et l'élasticité.

La résistance à la traction peut être mesurée par le palier à 15% d'extension. Le palier à 15% d'extension est la force qu'il faut appliquer à un cheveu mouillé d'une longueur donnée pour l'allonger de façon pemanente de 15%. Plus cette force est élevée, plus le cheveu est élastique et résistant.

Pour lutter contre l'agression de la kératine des cheveux par la lumière, on a déjà proposé d'utiliser certaines substances susceptibles de filtrer les radiations lumineuses, comme l'acide 2-hydroxy-4-méthoxy benzophénone-5-sulfonique ou ses sels (brevet français n° 2 627 085) ou l'acide 4-(2-oxo-3-bornylidène méthyl) benzène sulfonique ou ses sels (demande de brevet européen n° 0329032).

La demanderesse vient de découvrir, de façon tout à fait surprenante, que la lactoferrine, connue comme agent anti-radicaux libres par les demandes de brevet français n° 2 596 986 et 2 641 696, pouvait préserver les propriétés mécaniques des cheveux, et notamment leur résistance à la traction et leur élasticité, contre la dégradation par la lumière. Cette propriété a pu être mise en évidence par exposition en lumière naturelle (milieu ensoleillé) et en lumière artificielle (émetteur au xénon d'un appareil de vieillissement accéléré du type SUNTEST HANAU).

La présente invention a donc pour objet l'utilisation de la lactoferrine en tant qu'agent de protection des propriétés mécaniques des cheveux, et essentiellement de la résistance à la traction, contre la dégradation provoquée par les agressions atmosphériques, et en particulier par la lumière.

La lactoferrine est une protéine existant dans le lait. Elle peut être extraite et purifiée à partir du lait au moyen de techniques classiques de la biochimie préparative telles que des précipitations au sulfate d'ammonium, des ultrafiltrations ou des chromatographies d'échange d'ions.

Selon un mode de réalisation préféré de l'invention, la lactoferrine est utilisée sous forme d'une association de lactoferrine, de 8-hydroxyxanthine et de 2-thioxanthine.

On peut par exemple utiliser une solution aqueuse contenant ces trois composants dans les proportions en poids suivantes :
- lactoferrine : 0,2 à 2%
- 8-hydroxyxanthine : 0,01 à 0,1%
- 2-thioxanthine : 0,01 à 0,3 %.

Une telle composition est vendue par exemple sous la dénomination "INIFERINE" en solution aqueuse à 5,4% de matière sèche, dont 0,75 à 1% de lactoferrine, par la Société SEDERMA.

Selon l'invention, on utilise avantageusement la lactoferrine pour préserver les propriétés mécaniques des cheveux des agressions par la lumière en une quantité comprise entre 0,05 et 5% en poids et de préférence entre 0,05 et 3% en poids, dans un support cosmétiquement acceptable. Quand on utilise un mélange de lactoferrine, 8-hydroxyxanthine et 2-thioxanthine, dans les proportions indiquées ci-dessus, on peut donc être amené à enrichir ce mélange en lactoferrine dans le cas où la proportion de lactoferrine dépasse 2% en poids.

La lactoferrine peut être utilisée pour protéger les cheveux naturels ou sensibilisés. On entend par cheveux sensibilisés, des cheveux ayant subi un traitement de permanente, de coloration ou de décoloration.

Les compositions cosmétiques pour cheveux utilisées conformément à l'invention pour les protéger contre la dégradation par la lumière et contenant à titre de composés actifs, la lactoferrine ou son association ci-dessus définie, peuvent se présenter sous forme de solutions ou dispersions aqueuses ou hydroalcooliques, l'alcool étant le plus souvent un alcanol inférieur tel que l'éthanol ou l'isopropanol, épaissies ou non, de compositions huileuses, de crèmes, de gels, de dispersions vésiculaires de lipides amphipliles ioniques ou nonioniques, de mousses aérosols ou de sprays et contenir les adjuvants habituellement utilisés dans les compositions capillaires et adaptés à l'application envisagée.

Selon une variante de l'invention, les compositions cosmétiques contenant la lactoferrine, ou son association ci-dessus définie, peuvent en outre contenir des produits capables d'induire la dismutation des anions superoxydes en produisant de l'eau oxygénée et de l'oxygène, conne les enzymes superoxyde dismutases. On peut citer, à titre d'exemples, les enzymes superoxyde dismutases extraites de bactéries, de champignons, de sang ou encore la superoxyde dismutase d'origine narine vendue sous la marque "SUPERPHYCODISMUTASE" par la Société SECMA.

La présente invention a donc également pour objet une composition cosmétique pour cheveux comprenant de la lactoferrine ou son association ci-dessus définie, conjointement avec une enzyme superoxyde dismutase, dans un support cosmétiquement acceptable.

Les superoxyde dismutases sont utilisées en quantité comprise entre 0,01 et 5% en poids, et notamment entre 0,05 et 1% en poids, dans le support cosmétiquement acceptable.

Selon un aspect de l'invention, ces compositions constituent des compositions non rincées pour coiffer ou traiter la chevelure telles que des lotions, quels, crèmes, sprays ou mousses pour la mise en plis, pour le brushing, des compositions restructurantes ou des après-shampooings non rincés.

Selon un autre aspect de l'invention, ces compositions constituent des compositions rincées telles que des lotions, gels, crèmes ou mousses à appliquer avant ou après coloration, décoloration, permanente ou défrisage, après un shampooing ou entre l'étape de réduction et d'oxydation d'une permanente. Elles peuvent également constituer un shampooing.

Les compositions cosmétiques pour cheveux utilisées selon l'invention ont un pH compris entre 2 et 11, et de préférence entre 3 et 9.

Les compositions cosmétiques utilisées selon l'invention peuvent également renfermer des agents cosmétiques bien connus dans la technique sous réserve qu'ils n'altèrent pas eux-mêmes les propriétés mécaniques de la kératine des cheveux.

Les adjuvants ou agents cosmétiques généralement présents dans les compositions cosmétiques utilisées selon l'invention sont par exemple des épaississants, des polymères, des adoucissants, des conservateurs, des stabilisateurs de mousse, des huiles, des silicones, des agents de régulation de pH, des cires, des agents anti-gras, des agents séquestrants, des parfums, des colorants, des synergistes de mousses, des solvants organiques, des tensio-actifs et des propulseurs.

Les compositions cosmétiques utilisées selon l'invention peuvent contenir, dans un milieu aqueux ou hydroalcoolique, outre la lactoferrine ou son association, un polymère cationique, anionique, non-ionique, amphotère ou un mélange de tels polymères dans des quantités comprises généralement entre 0,1 et 3% en poids.

Ces compositions peuvent être des solutions aqueuses ou hydroalcooliques comprenant éventuellement des tensio-actifs; elles peuvent être aussi des émulsions ou des gels. Ces compositions peuvent également être pressurisées en aérosol sous forme de sprays ou de mousses.

Quand les compositions capillaires se présentent sous forme de mousses, elles contiennent un agent générateur de mousse en milieu aqueux ou hydroalcoolique, en présence d'un gaz propulseur.

Comme agent générateur de mousse, on peut utiliser des tensioactifs anioniques, non ioniques, cationiques, amphotères ou leurs mélanges, des polymères non-ioniques, anioniques, cationiques ou leurs mélanges, de l'alcool polyvinylique issu de polyacétate de vinyle hydrolysé dont le taux d'hydrolyse est égal ou inférieur à 97% comme décrit dans la demande de brevet FR 2 598 613.

Pour former une mousse, on peut utiliser une association de polymère cationique et de polymère anionique, au moins l'un des deux polymères étant moussant en solution aqueuse. De telles associations sont décrites dans le brevet FR 2 505 348.

Les gaz propulseurs utilisés pour pressuriser ces compositions destinées à former des mousses, sont présents dans des proportions ne dépassant pas 25% et de préférence 15% par rapport au poids total de la composition. On peut utiliser à titre de gaz propulseur le gaz carbonique, l'azote, le protoxyde d'azote, les hydrocarbures volatils tels que le butane, l'isobutane, le propane et leurs mélanges, les hydrocarbures chlorés et/ou fluorés non hydrolysables tels que ceux vendus sous les dénominations "FREON" ou "DYMEL" par la Société DU PONT de NEMOURS.

Lorsque la composition se présente sous forme de spray ou de laque, elle renferme dans un milieu alcoolique ou hydroalcoolique, une résine filmogène en présence éventuellement d'un gaz propulseur. On utilise de préférence, comme résine filmogène, un polymère anionique contenant des unités d'acides acrylique ou méthacrylique, d'acide crotonique ou d'acides α, β-dicarboxyliques insaturés.

Les agents propulseurs utilisés dans ces formulations sous forme de laques peuvent être choisis parmi les hydrocarbures volatils tels que le n-butane, le propane, l'isobutane ou leurs mélanges ou un mélange de ces hydrocarbures avec des hydrocarbures chlorés et/ou fluorés tels que les composés vendus sous la dénomination de "FREON" ou "DYMEL" par la Société DU PONT de NEMOURS et plus particulièrement les hydrocarbures fluorochlorés tels que le monofluorotrichlorométhane, le difluorodichlorométhane, le tétrafluorodichloroéthane ou les mélanges de ces derniers.

Ils peuvent également être choisis parmi les hydrocarbures chlorés et/ou fluorés susdécrits et leurs mélanges, le diméthyléther, le gaz carbonique ou le protoxyde d'azote.

La phase propulsive, dans ces compositions de laque représente 30 à 80% du poids total de la composition pressurisée.

Quand les compositions capillaires se présentent sous forme de gels, elles contiennent des épaississants en présence ou non de solvants.

Les épaississants peuvent être l'alginate de sodium, la gomme arabique ou la gomme de xanthane ou des dérivés cellulosiques tels que la métlyl cellulose, l'hydroxymétlyl cellulose, l'hydroxyéthyl cellulose, l'hydroxypropyl cellulose, la carboxyméthyl cellulose ou des polymères carboxyliques tels que les "Carbopol". On peut également obtenir un épaississement des lotions par mélange de polyéthylène glycol et de stéarate ou distéarate de polyéthylène glycol ou par un mélange d'esters phosphoriques et d'amides. La concentration en épaississant peut varier de 0,1 à 30%, et de préférence de 0,2 à 15% en poids par rapport au poids total de la composition.

Quand les compositions capillaires de l'invention constituent des lotions restructurantes, elles contiennent des produits renforçant la chaîne kératinique de cheveux. A cette classe des produits appartiennent les dérivés méthylolés tels que ceux décrits dans les brevets français n° 1 527 085 et 1 519 979.

Quand les compositions capillaires selon l'invention constituent des shampooings, elles contiennent en outre de 3 à 30% de tensio-actif anionique, non-ionique, amphotère ou leur mélange.

La présente invention vise également un procédé de protection de la kératine des cheveux contre les agressions atmosphériques, et en particulier contre la lumière, consistant à appliquer sur les cheveux une quantité efficace de lactoferrine ou de l'association lactoferrine, 8-hydroxyxanthine et 2-thioxanthine, dans un support cosmétiquement acceptable, cette application étant éventuellement suivie d'un rinçage à l'eau.

Les exemples suivants illustrent l'invention sans pour autant la limiter.

Dans les exemples 1 à 4 ci-après, l'INIFERINE, vendue par la Société SEDERMA, est une solution aqueuse contenant 5,4% de matière sèche dont 0,9% de lactoferrine.

### EXEMPLE 1

On prépare un gel de coiffage protecteur ayant la composition suivante :

| | |
|---|---|
| - Acide polyacrylique réticulé vendu sous la dénomination "CARBOPOL 940" par la Société GOODRICH | 0,5 g |
| - Copolymère vinylpyrrolidone/acétate de vinyle vendu sous la dénomination "PVP/VA S 630" par la Société GAF | 0.2 g |
| - INIFERINE | 3,0g de matière sèche (dont 0,5g de lactoferrine) |
| - Alcool éthylique | 5,0g |
| - Triéthanolamine qs pH | 7 |
| - Eau qsp | 100g |

### EXEMPLE 2

On prépare une mousse protectrice ayant la composition suivante :

| | |
|---|---|
| - Copolymère de chlorure de diméthyl diallyl ammonium et d'acrylamide vendu sous la dénomination "MERQUAT 550" en solution aqueuse à 8% de matière active par la Société GAF | 0,1 g MA |
| - Copolymère de chlorure de diméthyl diallyl animonium et d'hydroxyéthylcellulose vendu sous la dénomination "CELQUAT LOR" (28-6873) par la Société GAF | 0,5 g |
| - Hydroxyéthylcellulose vendue sous la dénomination "NATROSOL 250 HHR" par la Société AQUALON | 0,2 g |
| - INIFERINE | 1,0g de matière sèche (dont 0,16g de lactoferrine) |
| - Acide lactique qs pH 5 | |
| - Eau qsp | 100g |

| **Conditionnement aérosol** : | |
|---|---|
| - Composition ci-dessus | 92,0 g |
| - Mélange ternaire de n-butane, isobutane >55%, propane vendu sous la dénomination "AEROGAZ 3,2N" par la Société ELF AQUITAINE | 8,0g |

### EXEMPLE 3

On prépare une lotion protectrice ayant la composition suivante :

| | |
|---|---|
| - INIFERINE | 5,0g de matière sèche (dont 0,83 g de lactoferrine) |
| - Glycérine | 1,0g |
| - Alcool éthylique | 4,0g |
| - Eau qsp | 100g |

### EXEMPLE 4

On prépare une lotion spray protectrice ayant la composition suivante :

| | |
|---|---|
| - INIFERINE | 0,5 g de matière sèche (dont 0,08 g de lactoferrine) |
| - Copolymère de méthylvinyléther et de monomaléate de butyle vendu en solution alcoolique à 50% de matière active sous la dénomination "GANTREZ ES 425" par la Société GAF | 0,2 g MA |
| - Triéthanolamine qs pH 7 | |
| - Alcool éthylique | 10,0g |
| - Eau qsp | 100g |

### EXEMPLE 5

On prépare un après-shampooing à rincer de composition suivante :

| | |
|---|---|
| - Lactoferrine | 2 g |
| - Mélange d'alcool cétylstéarylique et d'alcool cétylstéarylique oxyéthyléné à 33 moles d'oxyde d'éthylène vendu sous la dénomination "SINNOWAX AO" par la Société HENKEL | 2 g |
| - Alcool stéarylique | 1 g |
| - Alcool cétylique | 1 g |
| - Hydroxyéthylcellulose vendue sous la dénomination "NATROSOL 250 HHR" par la société AQUALON | 1 g |
| - Parfum, colorants, conservateur qs | |
| - Hydroxyde de sodium qs pH 5,5 | |
| - Eau qsp | 100g |

### EXEMPLE 6

| **Shampooing protecteur** : | |
|---|---|
| -Alkyl (C₁₂/C₁₄) éther sulfate de sodium oxyéthyléné à 2,2 moles d'oxyde d'éthylène | 15g MA |
| - Monoisopropanolamide d'acide de coprah | 1 g |
| - Di-stéarate de glycol | 2 g |
| - Lactoferrine | 1 g |
| - Colorant, parfum, conservateur qs | |
| - Eau qsp | 100g |
| Le pH est ajusté à 6,5 avec de la triéthanolamine. | |

### EXEMPLE 7

| **Lotion capillaire protectrice** : | |
|---|---|
| - Lactoferrine | 0,5 g |
| - Superoxyde dismutase | 0,1g |
| - Glycérine | 1 g |
| - Alcool éthylique | 10 g |
| - Eau qsp | 100g |

## Revendications

1. Utilisation de la lactoferrine pour préserver les propriétés mécaniques des cheveux, et notamment leur résistance à la traction et leur élasticité, de la dégradation par les agressions atmosphériques, et en particulier par la lumière.

2. Utilisation selon la revendication 1 de la lactoferrine en une quantité comprise entre 0,05 et 5% en poids, dans un support cosmétiquement acceptable.

3. Utilisation selon la revendication 1 de lactoferrine sous forme d'association lactoferrine, 8-hydroxyxanthine et 2-thioxanthine.

4. Utilisation selon la revendication 3 d'une solution aqueuse de lactoferrine, 8-hydroxyxanthine et 2-thioxanthine contenant 0,2 à 2% en poids de lactoferrine, 0,01 à 0,1% en poids de 8-hydroxyxanthine et 0,01 à 0,3% en poids de 2-thioxanthine.

5. Utilisation selon l'une quelconque des revendications 1 à 4 de la lactoferrine ou de son association ci-dessus définie conjointement avec une enzyme superoxyde dismutase, dans un support cosmétiquement acceptable.

6. Utilisation selon la revendication 5 d'une enzyme superoxyde dismutase en une quantité comprise entre 0,01 et 5% en poids, dans un support cosmétiquement acceptable, conjointement avec la lactoferrine ou son association ci-dessus définie.

7. Utilisation selon l'une quelconque des revendications 1 à 6 de la lactoferrine dans des compositions cosmétiques pour cheveux se présentant sous forme de solutions ou dispersions aqueuses ou hydroalcooliques, épaissies ou non, de compositions huileuses, de crèmes, de gels, de dispersions vésiculaires de lipides amphiphiles ioniques ou non-ioniques, de mousses aérosols ou de sprays.

8. Utilisation selon la revendication 7 de la lactoferrine dans des compositions cosmétiques pour cheveux contenant en outre au moins un adjuvant cosmétique choisi parmi les épaississants, les polymères, les adoucissants, les conservateurs, les stabilisateurs de mousse, les huiles, les silicones, les agents de régulation de pH, les cires, les agents anti-gras, les agents séquestrants, les parfums, les colorants, les synergistes de mousses, les solvants organiques, les tensio-actifs et les propulseurs.

9. Utilisation selon la revendication 8 de la lactoferrine dans des compositions cosmétiques pour cheveux contenant au moins un polymère cationique, anionique, non ionique, amphotère ou un mélange de tels polymères dans des quantités compises entre 0,1 et 3% en poids.

10. Utilisation selon l'une quelconque des revendications 1 à 9 de la lactoferrine dans des compositions cosmétiques sous forme de mousse, contenant en milieu aqueux ou hydroalcoolique, en présence d'un gaz propulseur, un agent générateur de mousse choisi parmi les tensio-actifs anioniques, non-ioniques, cationiques, amphotères ou leurs mélanges, les polymères non-ioniques, anioniques, cationiques ou leurs mélanges, l'alcool polyvinylique issu de polyacétate de vinyle hydrolysé dont le taux d'hydrolyse est égal ou inférieur à 97%.

11. Utilisation selon la revendication 10 de la lactoferrine dans une composition cosmétique sous forme de mousse, contenant une association d'un polymère cationique et d'un polymère anionique en milieu aqueux ou hydroalcoolique, au moins l'un des deux polymères étant moussant en solution aqueuse, en présence d'un propulseur.

12. Utilisation selon l'une quelconque des revendications 1 à 9 de lactoferrine dans un spray ou une laque renfermant, dans un milieu alcoolique ou hydroalcoolique, éventuellement en présence d'un gaz propulseur, une résine filmogène constituée par un polymère anionique contenant des unités d'acide acrylique ou méthacrylique, d'acide crotonique ou d'acides α, β-dicarboxyliques insaturés.

13. Utilisation selon l'une quelconque des revendications 1 à 9 de lactoferrine dans un shampooing renfermant de 0,3 à 30% en poids d'un tensio-actif anionique, non-ionique, amphotère ou leur mélange.

14. Procédé de protection des propriétés mécaniques des cheveux, et notamment de leur résistance à la traction et de leur élasticité, contre les agressions atmosphériques, et en particulier contre la lumière, caractérisé par le fait qu'il consiste à appliquer sur les cheveux une quantité efficace de lactoferrine, dans un support cosmétiquement acceptable.

15. Procédé selon la revendication 14, caractérisé par le fait qu'il consiste à appliquer sur les cheveux de la lactoferrine dans une proportion comprise entre 0,05 et 5% en poids dans un support cosmétiquement acceptable.

16. Procédé selon la revendication 14 ou 15, caractérisé par le fait qu'il consiste à appliquer sur les cheveux une association de lactoferrine, 8-hydroxyxanthine et 2-thioxanthine.

17. Procédé selon la revendication 16, caractérisé par le fait qu'il consiste à appliquer sur les cheveux, une solution aqueuse de lactoferrine, 8-hydroxyxanthine et 2-thioxanthine contenant 0,2 à 2% en poids de lactoferrine, 0,01 à 0,1% en poids de 8-hydroxyxanthine et 0,01 à 0,3% en poids de 2-thioxanthine.

18. Procédé selon l'une quelconque des revendications 14 à 17, caractérisé par le fait qu'il consiste à appliquer sur les cheveux la lactoferrine ou son association ci-dessus définie, conjointement avec une enzyme superoxyde dismutase, dans un support cosmétiquement acceptable.

19. Procédé selon la revendication 18, caractérisé par le fait qu'il consiste à appliquer sur les cheveux 0,01 à 5% en poids d'enzyme superoxyde dismutase, dans un support cosmétiquement acceptable, conjointement avec la lactoferrine ou son association ci-dessus définie.

20. Procédé selon l'une quelconque des revendications 14 à 19, caractérisé par le fait que l'application sur les cheveux n'est pas suivie d'un rinçage.

21. Procédé selon l'une quelconque des revendications 14 à 19, caractérisé par le fait que l'application sur les cheveux est suivie d'un rinçage à l'eau.

22. Composition cosmétique pour cheveux, caractérisée par le fait qu'elle comprend de la lactoferrine ou son association ci-dessus définie, conjointement avec une enzyme superoxyde dismutase, dans un support cosmétiquement acceptable.

23. Composition cosmétique pour cheveux selon la revendication 22, caractérisée par le fait qu'elle comprend 0,05 à 5% en poids de lactoferrine et 0,01 à 5% en poids d'enzyme superoxyde dismutase, dans un support cosmétiquement acceptable.

## Claims

1. Use of lactoferrin for preserving the mechanical properties of hair, and in particular its tensile strength and its elasticity , from damage by environmental attacks, and in particular by light.

2. Use according to Claim 1 of lactoferrin in an amount of between 0.05 and 5 % by weight , in a cosmetically acceptable carrier.

3. Use according to Claim 1 of lactoferrin in the form of a combination , lactoferrin , 8-hydroxyxanthine and 2-thioxanthine.

4. Use according to Claim 3 of a combination of lactoferrin, 8-hydroxyxanthine and 2-thioxanthine in proportions of 0.2 to 2 % by weight of lactoferrin, 0.01 to 0.1 % by weight of 8-hydroxyxanthine and 0.01 to 0.3 % by weight of 2-thioxanthine .

5. Use according to any one of Claims 1 to 4 of lactoferrin or the above-defined combination, together with a superoxide dismutase enzyme, in a cosmetically acceptable carrier.

6. Use according to Claim 5 of a superoxide dismutase enzyme in an amount of between 0.01 and 5 % by weight, in a cosmetically acceptable carrier, together with lactoferrin or the above-defined combination thereof.

7. Use according to any one of Claims 1 to 6 of lactoferrin in cosmetic compositions for the hair which are provided in the form of aqueous or aqueous-alcoholic solutions or dispersions which are thickened or unthickened , oily compositions, creams, gels, vesicular dispersions of ionic or nonionic amphilic lipids, aerosol foams or sprays.

8. Use according to Claim 7 of lactoferrin in cosmetic compositions for the hair containing in addition at least one cosmetic adjuvant chosen from thickeners, polymers,demulcents,preservatives ,foam stabilisers, oils, silicones, pH-regulating agents, waxes, anti-fat agents, sequestering agents, perfumes, colorants, foam synergists, organic solvents, surface-active agents and propellants.

9. Use according to Claim 8 of lactoferrin in cosmetic compositions for the hair containing at least one cationic,anionic, nonionic or amphoteric polymer or a mixture of such polymers in amounts of between 0.1 and 3 % by weight.

10. Use according to any one of Claims 1 to 9 of lactoferrin in cosmetic compositions in foam form , containing in an aqueous or aqueous-alcoholic medium, in the presence of a propellant gas, a foam generating agent chosen from anionic, nonionic, cationic or amphoteric surface-active agents or mixtures thereof , nonionic, anionic or cationic polymers or mixtures thereof, polyvinyl alcohol derived from hydrolysed polyvinyl acetate whose hydrolysis level is not more than 97 %.

11. Use according to Claim 10 of lactoferrin in a cosmetic composition in foam form, containing a combination of a cationic polymer and an anionic polymer in aqueous or aqueous-alcoholic medium, at least one of the two polymers being foaming in aqueous solution, in the presence of a propellant.

12. Use according to any one of Claims 1 to 9 of lactoferrin in a spray or a lacquer containing, in an alcoholic or aqueous-alcoholic medium , optionally in the presence of a propellant gas, a film-forming resin consisting of an anionic polymer containing unsaturated acrylic or methacrylic acid, crotonic acide or -dicarboxylic acid units.

13. Use according to any one of Claims 1 to 9 of lactoferrin in a schampoo containg 0.3 to 30 % by weight of an anionic, nonionic or amphoteric surface-active agent or a mixture thereof.

14. Method of protecting the mechanical properties of hair, and in particular their tensile strength and their elasticity , against environmental attacks, and in particular against light, characterised in that it consists in applying to the hair an effective amount of lactoferrin, in a cosmetically acceptable carrier.

15. Method according to Claim 14, characterised in that it consists in applying to the hair lactoferrin in a proportion of between 0.05 and 5 % by weight in a cosmetically accepable carrier.

16. Method according to Claim 14 or 15, characterised in that it consists in applying to the hair a combination of lactoferrin, 8-hydroxyxanthine and 2-thioxanthine.

17. Method according to Claim 16, characterised in that it consists in applying to the hair , a combination of lactoferrin, 8-hydroxyxanthine and 2-thioxanthine in proportions of 0.2 to 2 % by weight of lactoferrin , 0.01 to 0.1 % by weight of 8-hydroxyxanthine and 0.01 and 0.3 % by weight of 2-thioxanthine.

18. Method according to any one of Claims 14 to 17, characterised in that it consists in applying to the hair lactoferrin or the above-defined combination thereof , together with a superoxide dismutase enzyme, in a cosmetically acceptable carrier.

19. Method according to Claim 18, characterised in that it consists in applying to the hair 0.01 to 5 % by weight of superoxide dismutase enzyme, in a cosmetically acceptable carrier, together with lactoferrin or the above-defined combination thereof.

20. Method according to any one of Claims 14 to 19 , characterised in that the application to the hair is not followed by rinsing.

21. Method according to any one of Claims 14 to 19, characterised in that the application to the hair is followed by rinsing with water.

22. Cosmetic composition for the hair, characterised in that it contains lactoferrin or the above-defined combination thereof, together with a superoxide dismutase enzyme , in a cosmetically acceptable carrier.

23. Cosmetic composition for the hair according to Claim 22, characterised in that: it contains 0.05 to 5 % by weight of lactoferrin and 0.01 to 5 % by weight of superoxide dismutase enzyme , in a cosmetically acceptable carrier.

## Patentansprüche

1. Verwendung von Lactoferrin zur Bewahrung der mechanischen Eigenschaften der Haare und insbesondere von deren Zugbeständigkeit und Elastizität vor einem Abbau durch Angriffe aus der Atmosphäre, und insbesondere durch Lichteinwirkung.

2. Verwendung gemäß Anspruch 1 des Lactoferrins in einer Menge von 0,05 bis 5 Gew.% in einem kosmetisch geeigneten Trägermedium.

3. Verwendung gemäß Anspruch 1 von Lactoferrin in Form einer Mischung aus Lactoferrin, 8-Hydroxyxanthin und 2-Thioxanthin.

4. Verwendung gemäß Anspruch 3 einer wässrigen Lösung von Lactoferrin, 8-Hydroxyxanthin und 2-Thioxanthin, welche 0,2 bis 2 Gew.% Lactoferrin, 0,01 bis 0,1 Gew.% 8-Hydroxyxanthin und 0,01 bis 0,3 Gew.% 2-Thioxanthin enthält.

5. Verwendung gemäß jedem Anspruch 1 bis 4 von Lactoferrin oder seiner oben definierten Mischung zusammen mit einem Superoxid-Dismutase-Enzym in einem kosmetisch geeigneten Trägermedium.

6. Verwendung gemäß Anspruch 5 eines Superoxid-Dismutase-Enzyms in einer Menge von 0,01 bis 5 Gew.%, in einem kosmetisch geeigneten Trägermedium, zusammen mit dem Lactoferrin oder seiner oben definierten Mischung.

7. Verwendung gemäß jedem Anspruch 1 bis 6 von Lactoferrin in kosmetischen Zusammensetzungen für Haare, welche in Form von wässrigen oder hydroalkcholischen, verdickten oder nichtverdickten Lösungen oder Dispersionen, öligen Zusammensetzungen, Creme-Produkten, Gelen, bläschenartigen Dispersionen aus amphiphilen ionischen oder nicht-ionischen Lipiden, Aerosol-Schäumen oder von Spray-Produkten vorliegen.

8. Verwendung gemäß Anspruch 7 von Lactoferrin in kosmetischen Zusarmensetzungen für Haare, welche ausserdem mindestens einen kosmetischen Hilfsstoff enthalten, ausgewählt aus Verdickungsmitteln, Polymeren, Weichmachern, Konservierungsmitteln, Schaumstabilisatoren, Ölen, Silikonen, pH-Reguliermitteln, Wachsen, Antifettmitteln, Sequestriermitteln., Parfüm-Produkten, Färbemitteln, Schaumsynergisten, organischen Lösungsmitteln, oberflächenaktiver. Mitteln und aus Treibmitteln.

9. Verwendung gemäß Anspruch 8 von Lactoferrin in kosmetischen Zusammensetzungen für Haare, welche mindestens ein kationisches, anionisches, nicht-ionisches oder amphoteres Polymer oder eine Mischung solcher Polymere in Mengen von 0,1 bis 3 Gew.% enthalten.

10. Verwendung gemäß jedem Anspruch 1 bis 9 von Lactoferrin in kosmetischen Zusammensetzungen in Form eines Schaums, die in wässrigem oder hydroalkoholischen Milieu, in Gegenwart eines gasförmigen Treibmittels, ein Schaumerzeugungsmittel enthalten, ausgewählt aus anionischen, nicht-ionischen, kationischen oder amphoteren oberflächenaktiven Mitteln oder aus deren Mischungen, aus nicht-ionischen, anionischen oder kationischen Polymeren oder aus deren Mischungen, aus Polyvinylalkohol aus hydrolysiertem Polyvinylacetat mit einem Hydrolysegrad von gleich oder weniger 97%.

11. Verwendung gemäß Anspruch 10 von Lactoferrin in der kosmetischen Zusammensetzung in Form eines Schaumes, welche eine Mischung aus einem kationischen Polymer und einem anionischen Polymer in wässrigem oder hydroalkoholischen Milieu, wobei mindestens eines der beiden Polymere in wässriger Lösung schäumend ist, in Gegenwart eines Treibmittels enthält.

12. Verwendung gemäß jedem Anspruch 1 bis 9 von Lactoferrin in einem Spray oder Lack, welche, in alkoholischem oder hydroalkoholischen Milieu, gegebenenfalls in Gegenwart eines gasförmigen Treibmittels, ein filmbildendes Harz aus einem anionischen Polymer mit Einheiten aus Acryl- oder Methacrylsäure, Krotonsäure oder ungesättigten α,β-Dicarboxylsäuren enthalten.

13. Verwendung gemäß jedem Anspruch 1 bis 9 von Lactoferrin in einem Shampoo, das 0,3 bis 30 Gew.% eines anionischen, nicht-ionischen oder amphoteren oberflächenaktiven Mittels oder von deren Mischung enthält.

14. Verfahren zum Schutz der mechanischen Eigenschaften der Haare, und insbesondere von deren Zugbeständigkeit und Elastizität, vor Angriffseinwirkungen aus der Atmosphäre und insbesondere vor Licht,
dadurch **gekennzeichnet**, daß man auf die Haare eine wirksame Menge von Lactoferrin, in einem kosmetisch geeigneten Trägermedium, aufbringt.

15. Verfahren gemäß Anspruch 14,
dadurch **gekennzeichnet**, daß
man auf die Haare Lactoferrin in einem Mengenanteil von 0,05 bis 5 Gew% in einem kosmetisch geeigneten Trägermedium aufbringt.

16. Verfahren gemaß Anspruch 14 oder 15,
dadurch **gekennzeichnet**, daß
man auf die Haare eine Mischung aus Lactoferrin, 8-Hydroxyxanthin und 2-Thioxanthin aufbringt.

17. Verfahren gemäß Anspruch 16,
dadurch **gekennzeichnet**, daß
man auf die Haare eine wässrige Lösung von Lactoferrin, 8-Hydorxyxanthin und 2-Thioxanthin aufbringt, welche 0,2 bis 2 Gew.% Lactoferrin, 0,01 bis 0,1 Gew.% 8-Hydroxyxanthin und 0,01 bis 3 Gew.% 2-Thioxanthin enthält.

18. Verfahren gemäß jedem Anspruch 14 bis 17,
dadurch **gekennzeichnet**, daß
man auf die Haare Lactoferrin oder seine oben definierte Mischung, zusammen mit einem Superoxid-Dismutase-Enzym, in einem kosmetisch geeigneten Trägermedium aufbringt.

19. Verfahren gemäß Anspruch 18,
dadurch **gekennzeichnet**, daß
man auf die Haare 0,01 bis 5 Gew.% Superoxid-Dismutase-Enzym, in einem kosmetisch geeigneten Trägermedium, zusammen mit dem Lactoferrin oder seiner oben definierten Mischung aufbringt.

20. Verfahren gemäß jedem Anspruch 14 bis 19,
dadurch **gekennzeichnet**, daß
nach der Aufbringung auf die Haare nicht gespült wird.

21. Verfahren gemäß jedem Anspruch 14 bis 19,
dadurch **gekennzeichnet**, daß
nach der Aufbringung auf die Haare mit Wasser gespült wird.

22. Kosmetische Zusammensetzung für Haare,
dadurch **gekennzeichnet**, daß
sie Lactoferrin oder seine oben definierte Mischung, zusammen mit einem Superoxid-Dismutase-Enzym, in einem kosmetisch geeigneten Trägernedium enthält.

23. Kosmetische Zusammensetzung für Haare gemäß Anspruch 22,
dadurch **gekennzeichnet**, daß
sie 0,05 bis 5 Gew.% Lactoferrin und 0,01 bis 5 Gew.% Superoxid-Dismutase-Enzym, in einem kosmetisch geeigneten Trägermedium, enthält.
